(19) **Europäisches Patentamt / European Patent Office / Office européen des brevets**

(11) **EP 2 962 634 B1**

(12) **EUROPEAN PATENT SPECIFICATION**

(45) Date of publication and mention
of the grant of the patent:
**31.01.2018 Bulletin 2018/05**

(21) Application number: **14751919.3**

(22) Date of filing: **13.02.2014**

(51) Int Cl.:
*A61B 5/04* (2006.01)     *A61B 5/048* (2006.01)
*A61B 5/0484* (2006.01)

(86) International application number:
**PCT/KR2014/001201**

(87) International publication number:
**WO 2014/126406 (21.08.2014 Gazette 2014/34)**

(54) **METHOD AND APPARATUS FOR MEASURING ANESTHETIC DEPTH**

VERFAHREN UND VORRICHTUNG ZUR MESSUNG DER ANÄSTHETISCHEN TIEFE

PROCÉDÉ ET APPAREIL DE MESURE D'UNE PROFONDEUR D'ANESTHÉSIE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **18.02.2013 KR 20130016794**

(43) Date of publication of application:
**06.01.2016 Bulletin 2016/01**

(73) Proprietor: **Charm Engineering Co., Ltd.
Gyeonggi-do 449-884 (KR)**

(72) Inventors:
• **CHANG, Ho-Jong
Daejeon 302-753 (KR)**
• **KIM, Eung Hwi
Daejeon 305-308 (KR)**
• **PARK, Sang Hyun
Gongju-si
Chungcheongnam-do 314-924 (KR)**
• **HONG, Seung Kyun
Seoul 110-043 (KR)**
• **KIM, Kwang Moo
Seongnam-si
Gyeonggi-do 463-892 (KR)**

(74) Representative: **Michalski Hüttermann & Partner
Patentanwälte mbB
Speditionstraße 21
40221 Düsseldorf (DE)**

(56) References cited:
EP-A1- 0 013 183     JP-A- 2006 255 134
KR-A- 20110 116 519     KR-A- 20120 009 610
KR-A- 20120 049 337     US-A1- 2005 137 494
US-A1- 2006 135 880     US-A1- 2009 275 853

• Rogean Rodrigues Nunes ET AL: "Entropia
Espectral: Um Novo Método para Adequação
Anestésica * Spectral Entropy: A New Method for
Anesthetic Adequacy", , 1 January 2004
(2004-01-01), XP055307590, Retrieved from the
Internet:
URL:http://www.scielo.br/pdf/rba/v54n3/en_
v54n3a13.pdf [retrieved on 2016-10-04]
• PUTHANKATTIL SUBHA D ET AL: "EEG Signal
Analysis: A Survey", JOURNAL OF MEDICAL
SYSTEMS, KLUWER ACADEMIC
PUBLISHERS-PLENUM PUBLISHERS, NE, vol.
34, no. 2, 6 December 2008 (2008-12-06), pages
195-212, XP019791847, ISSN: 1573-689X

EP 2 962 634 B1

**Description**

**TECHNICAL FIELD**

The present invention relates to a method for measuring an anesthetic depth, and more particularly, to a method and an apparatus for measuring an anesthetic depth, capable of providing an accurate measurement value of an anesthetic depth despite a change in an anesthetic condition, providing anesthetic depth information timely according to a change in an anesthetic condition by significantly improving a tracking speed, and having compatibility with a conventional anesthetic depth analyzing apparatus, thereby having high utilization.

**BACKGROUND ART**

Generally, in the field of medical practice including an operation and a treatment, when pain is applied to a subject, neurotransmission is blocked through anesthesia so that the pain is removed or reduced. During an operation for a severe disease or symptom, general anesthesia is performed, and a patient under general anesthesia should be continuously observed. An anesthetic state of a patient should be checked by sensing an anesthetic depth, and there is a problem. While an operation should be performed under sufficient anesthesia, there is a problem in that a patient suffers from mental pain due to awakening during the operation.

Accordingly, during an operation, an anesthetic depth should be continuously measured, and a method for observing a clinical aspect and a method for analyzing a bioelectric signal have been mainly used as a method for measuring an anesthetic depth. The method for analyzing a bioelectric signal includes a method for measuring and analyzing brainwaves so as to evaluate an effect of an anesthetic agent on the central nervous system, and there are also various kinds of monitoring apparatuses to which a method of using brainwaves is applied. The reason there are various kinds of anesthetic depth monitoring apparatuses using brainwaves is that the respective apparatuses have different algorithms for analyzing and evaluating brainwaves.

Rogean Rodrigues Nunes ET AL: "Spectral Entropy: A New Method for Anesthetic Adequacy", Rev Bras Anestesiol, Vol.54, Nr. 3, pp. 402-422, shows a method for measuring an anesthetic depth, by dividing an EEG signal into multiple epoch signals, extracting a Shannon entropy calculation value, and extracting a spectra entropy calculation value.

Currently, a bispectral index (hereinafter, referred to as "BIS") analyzing apparatus is most popularly used as an anesthetic depth monitoring apparatus. The BIS analyzing apparatus, is one of the apparatuses in which a brainwave-based anesthetic depth measuring technique is developed and adopted for the first time therein, displays an anesthetic depth as "BIS" to be digitized within a range of 0-100, and verifies the clinical reliability of BIS by comparing BIS with a conventional anesthetic depth measuring standard or with an index calculated in another anesthetic depth instrument.

With a conventional anesthetic depth monitoring apparatus including such as BIS analyzing apparatus, a user, who is an anesthetic depth clinical subject or an anesthetic depth monitor, is unable to improve or change brainwave analyzing algorithms of instruments, so that an algorithm suitable for patient's characteristics of a patient may not be applied and accordingly an anesthetic depth of the patient cannot be accurately monitored. Furthermore, since the details of analyzing algorithms installed in instruments are not disclosed, the apparatus is not suitable for a clinical anesthetic depth study and there are many difficulties in proving an algorithm error.

Moreover, an anesthetic depth monitoring apparatus such as the BIS analyzing apparatus has a problem in that an anesthetic state of a patient is unable to be rapidly sensed because a speed for tracking a rapid change in an anesthetic state is slow.

Patent Document 1 relates to a system and a method for measuring a brain activity and an anesthetic depth through a brainwave signal analysis, wherein values may be very accurately calculated compared to a conventional spectrum analysis, wavelet analysis, or entropy analysis, although the structure of a basic algorithm is very simple.

(Patent Document 1) Korean Patent Application Laid-open Publication No. 2012-0131027 (publicized on December 4, 2012)

**DISCLOSURE OF THE INVENTION**

**TECHNICAL PROBLEM**

To resolve the above-described problem, an object of the present invention is to provide a method and an apparatus for measuring an anesthetic depth, capable of providing an accurate measurement value of an anesthetic depth despite a change in an anesthetic condition, providing anesthetic depth information timely despite a rapid change in an anesthetic state, and having compatibility with a conventional anesthetic depth analyzing apparatus.

## TECHNICAL SOLUTION

To resolve the above-described problem, a method for measuring an anesthetic depth according to the present invention includes: dividing, by an epoch dividing part, an EEG signal into multiple epoch signals in time units, extracting, by a counting part, a CAI calculation value (CAI) by counting the number of points in epoch of a value higher than a determined critical value, extracting, by a Shannon entropy calculating part, a Shannon entropy calculation value (ShEn) by performing a Shannon entropy calculation from the EEG signal, and extracting, by a spectra entropy calculation part, a spectra entropy calculation value (SpEn) by performing a spectra entropy calculation; extracting, by a modified Shannon entropy calculating part, a modified Shannon entropy calculation value (MshEn) by multiplying the Shannon entropy calculation value (ShEn) and spectra entropy calculation value (SpEn); and extracting, by a CAI extracting part, an anesthetic depth index (MsCAI) by performing a logical operation on the modified Shannon entropy calculation value (MshEn) and CAI calculation value (CAI).

According to a preferable embodiment of the present invention, the extracting of the anesthetic depth index (MsCAI) includes extracting the anesthetic depth index (MsCAI) by multiplying the modified Shannon entropy calculation value (MshEn) by a first constant (L) and multiplying the CAI calculation value (CAI) by a second constant (U) to be summed up according to the equation MsCAI = L * MshEn + U * CAI (L = 0.243, $0.65 \leq U \leq 0.74$).

According to a preferable embodiment of the present invention, the extracting of the Shannon entropy calculation value (ShEn) and spectra entropy calculation value (SpEn) includes: performing low frequency band pass filtering on the EEG signal; performing high frequency band pass filtering on the signal obtained after the low frequency band pass filtering; generating a first epoch signal by dividing the signal obtained after the low frequency band pass filtering by predetermined time units; generating a second epoch signal by dividing the signal obtained after the high frequency band pass filtering by predetermined time units; removing noise from the first epoch signal; and performing normalization by dividing the second epoch signal by a root mean square value of the epoch signal having noise removed therefrom.

According to a preferable embodiment of the present invention, the method includes: calculating a power spectrum density of a frequency component outputted by performing high speed Fourier transformation on the epoch signal with noise removed; and extracting the spectra entropy calculation value by performing a spectra entropy calculation from the power spectrum density.

According to a preferable embodiment of the present invention, the method includes extracting the Shannon entropy calculation value by performing a Shannon entropy calculation from the normalized signal.

According to a preferable embodiment of the present invention, the method includes performing high speed Fourier transformation on the normalized signal and extracting the spectra entropy calculation value the power spectrum density.

According to a preferable embodiment of the present invention, the method includes calculating the critical value by performing discrete Fourier transformation on the normalized signal to be multiplied at least twice, summed up to a predetermined frequency band, and multiplied by a constant.

According to a preferable embodiment of the present invention, the method includes extracting the CAI calculation value by counting the number of points which are larger than the critical value, dividing the counted number by the total number of points of the epoch signal, and multiplying the divided value by a predetermined value.

According to another embodiment of the present invention, an apparatus for measuring an anesthetic depth according to the present invention includes: a CAI extracting part for configured to divide an EEG signal in a time section to generate an epoch signal, setting a predetermined critical value from the epoch signal, and extracting a cortical activity index (CAI) calculation value (CAI) by counting the number of points in an epoch signal exceeding the critical value; a modified Shannon entropy extracting part configured to output a modified Shannon entropy calculation value (MshEn) by multiplying a Shannon entropy calculation value (ShEn) calculated from the EEG signal and a spectra entropy calculation value (SpEn) calculated from the EEG signal; and an MsCAI extracting part configured to extract an anesthetic depth index (MsCAI) by performing a logical operation on the modified Shannon entropy calculation value (MshEn) and CAI calculation value (CAI).

According to a preferable embodiment of the present invention, the MsCAI extracting part extracts the anesthetic depth index (MsCAI) by multiplying the modified Shannon entropy calculation value (MshEn) by a first constant (L) and multiplying the CAI calculation value (CAI) by a second constant (U) to be summed up according to the equation MsCAI = L * MshEn + U * CAI (L = 0.243, $0.65 \leq U \leq 0.74$).

According to a preferable embodiment of the present invention, the CAI extracting part further includes a critical value extracting part configured to fluidly change a constant multiplied by the critical value according to an anesthetic degree or a frequency band, so as to apply the anesthetic degree or frequency band to the critical value.

According to a preferable embodiment of the present invention, the critical value extracting part extracts the critical value by multiplying a value after discrete Fourier transformation performed on a normalized signal at least twice to be summed up to a predetermined frequency band and multiplied by a constant.

According to a preferable embodiment of the present invention, the CAI extracting part further includes a counter for configured to count the number of points which are larger than the critical value in the epoch signal and divide the counted number by the total number of points in the epoch signal.

According to a preferable embodiment of the present invention, the apparatus further includes: a first epoch dividing part configured to generate a first epoch signal by dividing a signal, which is obtained by performing low frequency band pass filtering on the EEG signal, by predetermined time units; and a second epoch dividing part configured to generate a second epoch signal by performing high frequency band pass filtering on the signal obtained after the low frequency band pass filtering and by dividing the resultant signal by predetermined time units.

According to a preferable embodiment of the present invention, the apparatus further includes: a noise removing part configured to remove noise through a wavelet technique from an output of the first epoch dividing part; a normalizing part configured to calculate a root mean square value of an output epoch signal of the noise removing part and divide an output epoch signal of the second epoch dividing part by the root mean square value; and a Shannon entropy calculating part configured to calculate Shannon entropy from an output of the normalizing part so as to output the Shannon entropy calculation value.

According to a preferable embodiment of the present invention, the apparatus further includes: a power spectrum calculating part configured to perform high speed Fourier transformation on an output of the noise removing part and output a power spectrum density; and a spectra entropy calculating part configured to output a spectra entropy calculation value by calculating spectra entropy from an output of the power spectrum calculating part.

## ADVANTAGEOUS EFFECTS

In the present invention, a fisher score is 74.6365, and an anesthetic degree may thus be more accurately measured compared to a conventional BIS anesthetic depth analyzing apparatus (fisher score 47.11).

A conventional problem in that when an anesthetic degree is rapidly changed, a reaction speed is low because of a low tracking speed of a conventional BIS technique, is solved, and therefore a change in a state of from an awakening state to an anesthetic state (hypnosis) can be accurately and timely detected by more rapidly reacting than a conventional anesthetic depth analyzing apparatus with a reaction speed, which is averagely 15 seconds faster.

In the present invention, through a Shannon entropy technique and a modified Shannon entropy technique improved from a spectra entropy technique, not only irregularity in a time domain and a frequency domain is applied but also the brainwave's own characteristics are applied through a combination with a CAI technique, thereby accurately and rapidly providing a measured value of an anesthetic state according to various anesthetic conditions.

The present invention has a high correlation of 0.9877 with a conventional BIS apparatus, high compatibility with a conventional BIS apparatus thereby, and high compatibility with a structure concomitantly used for the BIS apparatus, and ensures high utilization.

According to the present invention, a real-time process may be easily performed due to a simple algorithm, thereby more accurately capturing a change in a state during anesthesia.

The present invention may be applied to a medical instrument for evaluating an anesthetic depth and may also be applied to a brainwave signal processing-related instrument having a different signal treating technique.

## BRIEF DESCRIPTION OF THE DRAWINGS

FIG. 1 is a graph showing a change in brainwaves according to an anesthetic degree.
FIG. 2 illustrates an apparatus for measuring an anesthetic depth according to the present invention.
FIG. 3 shows an algorithm of a method for measuring an anesthetic depth according to the present invention.
FIG. 4 is a conceptual diagram of an error removing part illustrated in FIG. 2.
FIG. 5 illustrates a screen display part illustrated in FIG. 2.

## MODE FOR CARRYING OUT THE INVENTION

Hereinafter, specific embodiments for carrying out the present invention will be described with reference to the accompanying drawings. In the drawings, the dimensions of main parts are exaggerated and ancillary parts are omitted for clarity of illustration. Thus, the present invention should not be construed as limited to the drawings.

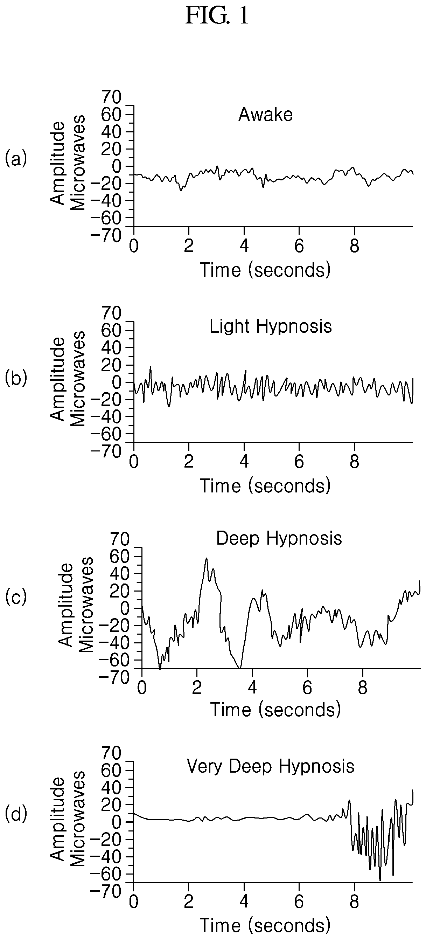

According to studies, it has been known that changes in characteristics of brainwaves during an operation have a great correlation with an anesthetic degree. Referring to FIG. 1, FIG. 1(a) shows measured brainwaves during an awake state, and brainwaves during an awake state have a small amplitude and a high frequency component. As a subject is entering into anesthesia (hypnosis), the amplitude becomes larger and the frequency component becomes lower as shown in FIGS. 1(b) and 1(c). When the subject is very deeply anesthetized, flat signals are outputted as shown in FIG. 1(d), and signals (burst suppression) having a high amplitude and a high frequency component are intermittently observed. Bio-signals such as changes in a heart rate, an electrocardiogram, and an electromyogram have a low direct correlation with an anesthetic degree. It is because various other reasons may affect a heart rate. On the other hand, unlike the correlation of a heart rate, it has been known through several researches that the characteristics of a brainwave signal have a direct correlation with an anesthetic degree of a patient when components of the brainwave signals are changed.

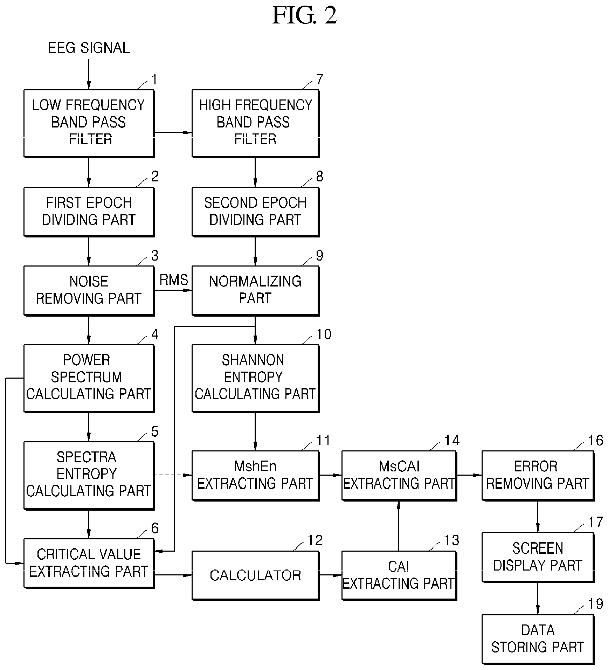

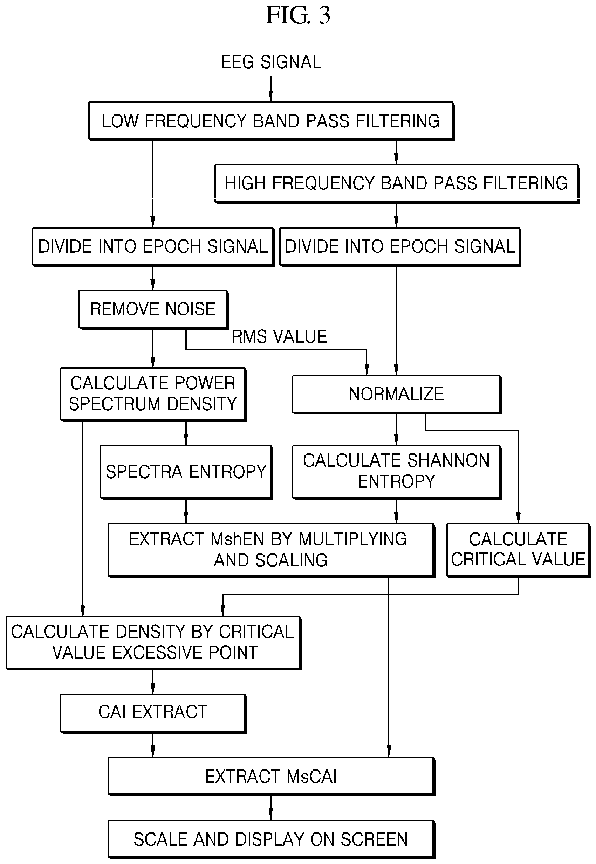

Hereinafter, referring to an apparatus for measuring an anesthetic depth illustrated in FIG. 2 and an algorithm for measuring an anesthetic depth illustrated in FIG. 3, description will be given of a method and an apparatus for measuring an anesthetic depth of the present invention.

The apparatus for measuring an anesthetic depth, according to the present invention, illustrated in FIG. 2 includes: a low frequency band pass filter 1; a high frequency band pass filter 7; a first epoch dividing part 2; a second epoch dividing part 8; a noise removing part; a normalizing part 9; a power spectrum calculating part 4; a Shannon entropy calculating part 10; a spectra entropy calculating part 5; a modified Shannon entropy (MsCAI) calculating part 11; a critical value extracting part 6; a counter 12; a cortical activity index (CAI) extracting part 13; a modified Shannon entropy with cortical activity index (MsCAI) extracting part 14; an error removing part 16; a screen display part 17; and a data storing part 19.

The low frequency band pass filter 1 removes electric noise of approximately 60 Hz or more from an electroencephalography (hereinafter, referred to as "EEG") signal through a patch adhered on the forehead of a subject. Although information that can be obtained from an EEG signal exists in various frequency bands, the low frequency band pass filter 1 performs an analysis by using a frequency between approximately 0-60 Hz and considers the signal having approximately 60 Hz or more as noise.

The high frequency band pass filter 7 performs high frequency band pass filtering on the signal passing through the low frequency band pass filter 1. Since a change in electrical power in a brainwave of a high frequency band has more direct correlation with an anesthetic depth, the output signal of the low frequency band pass filter 1 is filtered as a low frequency band signal.

The first epoch dividing part 2 divides the serially entering output signal of the low frequency band pass filter 1 into epoch signals (hereinafter referred to as a first epoch signal to be distinguished from an epoch signal of the second epoch diving part) each having a predetermined time unit (for examples, 16 seconds). The divided signal may overlap an adjacent signal. For example, the adjacent signal overlaps for a 15 second section, a divided signal is generated at every second to be outputted to the noise removing part 3.

The second epoch dividing part 8 has the same structure as the first epoch dividing part 2 but is different from the first epoch dividing part because an input signal is the output signal of the high frequency band pass filter 7. The second epoch dividing part 8 divides the output signal of the high frequency band pass filter 7 into epoch signals (hereinafter referred to as a second epoch signal to be distinguished from the epoch signal of the first epoch diving part) to be outputted to the normalizing part 9.

The noise removing part 3 removes noise (artifact) caused by the eyes and noise caused by the movement of a subject from the first epoch dividing part 2. The noise removing part 3, for example, performs a wavelet-based denoising technique.

The normalizing part 9 calculates a root mean square (hereinafter, referred to as "RMS") of an output epoch signal of the noise removing part 3 and divides an output epoch signal of the second epoch dividing part 8 by the RMS to be normalized.

The power spectrum calculating part 4 performs fast Fourier transformation (FFT) on the signal from which the noise has been removed, and power spectrum density (PSD) of an outputted frequency component is obtained. That is, a histogram of power (a squared value) of signal points is obtained.

The Shannon entropy calculating part 10, as shown in Equation 1, calculates Shannon Entropy of an output signal of the normalizing part 9 and outputs a Shannon entropy calculation value (ShEn). p(ai) is the number and frequency of factors corresponding to each block after the signal is divided by k (k>1) according to size. For example, assuming that the sizes of absolute values for each epoch signal are 1, 2, 2, 3, are divided into three portions, [0,5, 1.5], [1.5, 2.5], and [2.5, 3.5] and each block is respectively a1, a2, or a3, p(a2)=2/4. The calculation may be performed by applying irregularity of the histogram of the EEG signal, that is, irregularity of a time domain, by means of a Shannon entropy technique. Shannon entropy technique may be applied to a well-known art.

$$ShEn = \frac{-\sum_{i=1}^{k} p(a_i) \log p(a_i)}{\log k} \quad \ldots\ldots\ldots\ldots (Eq.\ 1)$$

The spectra entropy calculating part 5, as shown in Equation 2, performs a spectra entropy calculation on an output signal of the power spectrum calculating part 4 to extract a spectra entropy calculation value (SpEn). The spectra entropy calculation is similar to Shannon entropy but performs a Shannon entropy (ShEn) calculation by using a value obtained for the power spectrum density (PSD). Through the spectra entropy calculation, power spectrum of the EEG signal, that is, irregularity of a frequency domain, may be applied to an anesthetic depth index. A spectra entropy calculating technique may be applied to a well-known art.

$$SpEn = \frac{-\sum_{i=1}^{k} PSD(f_i) \log PSD(f_i)}{\log k} \quad \ldots\ldots\ldots\ldots\ldots \text{(Eq. 2)}$$

The modified Shannon entropy (hereinafter, referred to as "MshEn") extracting part 11 scales, in a predetermined range, and multiplies a Shannon entropy (ShEn) calculation value and a spectra entropy (SpEn) calculation value for each epoch signal. A scaling standard may be mapped onto a value, for example approximately 1-100, inversely proportional to an anesthetic degree of an output signal.

The critical value (threshold) extracting part 6, as shown in Equation 3, outputs a critical value (threshold) by squaring a value obtained after discrete Fourier transformation (DFT), summing up the value to a predetermined frequency band, and then multiplying a constant (K). A critical value for calculating the density of an impulse signal is calculated. Since the amplitude of the signal tends to increase when a subject is deeply anesthetized, the critical value is not fixed as one value but fluidly changed according to characteristics of the signal. Accordingly, a size of a low frequency band is set as the critical value. In Equation 3 below, K is an experimentally figured out coefficient, DFT is discrete Fourier transformation, $\overrightarrow{Xnor}$ is an output epoch signal of the normalizing part, and M is a 4 Hz band in a discrete time domain. The critical value extracting part 6 may compensate a bias error that is generated as the amplitude of the signal increases, by increasing the critical value when the low frequency band is large and the subject is deeply anesthetized.

$$Threshold = K \sum_{k=1}^{M} |DTF(\overrightarrow{Xnor})|^2 \quad \ldots\ldots\ldots\ldots\ldots \text{(Eq. 3)}$$

The counter 12 counts, in epoch, the number of points larger than the critical value extracted from the critical value extracting part 6 and dividing the counted number by the total number of points of epoch.

The cortical activity index (hereinafter, referred to as "CAI") extracting part 13 outputs a CAI calculation value (CAI) by scaling an output of the counter 12. The CAI extracting part 13 considers a signal emitted from a human brain cell as the sum of an impulse signal (or a peak signal), and the EEG signal as the sum of the impulse in a measuring point. Since brain activity is high in an awakening state, there are many impulse signals in the EEG signal. A CAI calculation value (CAI) is measured based on the fact that impulse is low during anesthesia.

The MsCAI extracting part 14 calculates an anesthetic depth index (MsCAI) by multiplying the modified Shannon entropy calculation value (MshEn) and CAI calculation value (CAI) by a predetermined coefficient obtained through an experiment. The anesthetic depth index (MsCAI) in the MsCAI extracting part 14 is calculated through, for example, Equation 4 and Equation 5.

$$MsCAI = ((A * ShEn) * (B * SpEn)) + U * CAI \quad \ldots\ldots\ldots\ldots\ldots \text{(Eq. 4)}$$

$$MsCAI = L * MshEn + U * CAI \quad (L = A * B) \ldots\ldots\ldots\ldots\ldots \text{(Eq. 5)}$$

Constant A, constant B, and constant U are experimentally figured out coefficients, which allow a fisher score to be optimized through repeated experiments and simulations. When the constant A, constant B, and constant U are changed in a 0.01 unit between 0 and 1, a change in a fisher score of an Ms CAI technique may be exemplified as Table 1. When A= 0.54, B= 0.45, and U= 0.71, the fisher score is 74.6365, which is the best. Moreover, when A= 0.54, B= 0.45, $0.65 \leq U \leq 0.74$, that is, when L (A*B) = 0.243 and $0.65 \leq U \leq 0.74$, the anesthetic depth index (MsCAI) of the present invention is increased by at least approximately 40% compared to a BIS algorithm. Referring to Table 1 and Table 2, it can be seen that the apparatus for measuring an anesthetic depth according to the present invention has a remarkably improved function compared to a conventional BIS technique.

[Table 1]

| Test No. | A | B | c | Fisher Score |
|---|---|---|---|---|
| 1 | 0.54 | 0.45 | 0.54 | 56.9255 |
| 2 | 0.54 | 0.45 | 0.55 | 58.589 |
| 3 | 0.54 | 0.45 | 0.56 | 60.337 |
| 4 | 0.54 | 0.45 | 0.57 | 59.2556 |
| 5 | 0.54 | 0.45 | 0.58 | 61.3602 |
| 6 | 0.54 | 0.45 | 0.59 | 62.3691 |
| 7 | 0.54 | 0.45 | 0.6 | 60.9082 |
| 8 | 0.54 | 0.45 | 0.61 | 62.3814 |
| 9 | 0.54 | 0.45 | 0.62 | 61.3606 |
| 10 | 0.54 | 0.45 | 0.63 | 63.1417 |
| 11 | 0.54 | 0.45 | 0.64 | 64.7456 |
| 12 | 0.54 | 0.45 | 0.65 | 69.5645 |
| 13 | 0.54 | 0.45 | 0.66 | 67.4387 |
| 14 | 0.54 | 0.45 | 0.67 | 69.2384 |
| 15 | 0.54 | 0.45 | 0.68 | 69.813 |
| 16 | 0.54 | 0.45 | 0.69 | 71.0052 |
| 17 | 0.54 | 0.45 | 0.7 | 70.8148 |
| 18 | **0.54** | **0.45** | **0.71** | **74.6356** |
| 19 | 0.54 | 0.45 | 0.72 | 68.5861 |
| 20 | 0.54 | 0.45 | 0.73 | 66.1987 |
| 21 | 0.54 | 0.45 | 0.74 | 67.3864 |
| 22 | 0.54 | 0.45 | 0.75 | 64.6739 |

[Table 2]

| Name of algorithm | Fisher score |
|---|---|
| MsCAI | 74.6365 |
| BIS | 47.11 |
| MshEn | 58.6232 |
| CAI | 42.4912 |

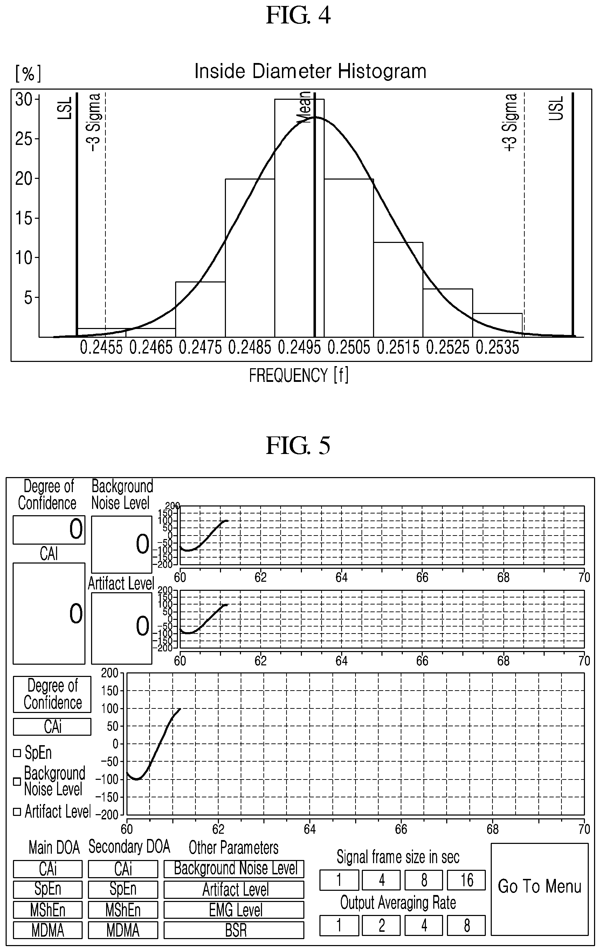

The error removing part 16 removes abnormal signals from an output of the MsCAI extracting part 14. It is highly likely that values resulted from noise are not correct values. A difference in a size from an adjacent point is represented as a histogram, as shown in FIG. 4, and then points corresponding to top 0.5% are determined to be inappropriate values. For the inappropriate values, the predetermined number (for example, approximately 15-30) of past values is averaged, and calculation is performed by adding higher weighting to recent values.

The screen display part 17, as illustrated in FIG. 5, displays an output of the error removing part 16 on a screen. When scaled signals are transmitted to monitoring software every second, the screen display part 17 displays an anesthetic depth on a screen by means of the monitoring software. At the same time, raw EEG signal and anesthetic depth index tendencies, signal quality, and other bio-signals (heart rate and electromyogram) are displayed together, thereby enabling an examiner to make an accurate determination.

The data storing part 19 stores measured anesthetic depth data, and the data may be extracted after an operation to be utilized as research materials in the future.

The apparatus for measuring an anesthetic depth according to the present invention may measure an anesthetic depth by applying irregularity of a time axis and irregularity of a frequency band by multiplying the Shannon entropy calculation value and spectra calculation value, so that the fisher score is higher and a reaction speed according to an anesthetic degree is higher than those obtained through the Shannon entropy calculation technique or spectra entropy calculation technique. Moreover, an optimal anesthetic depth analysis coefficient is extracted through an experiment by respectively weighting the modified Shannon entropy technique and the CAI technique having characteristics of brainwaves applied thereto, wherein the Shannon entropy calculation technique and spectra entropy calculation technique are multiplied, and irregularity in the time domain and frequency domain may be applied to the modified Shannon entropy technique. As a result, referring to Table 2, the fisher score of the MsCAI technique according to the present invention is greatly improved to 74.6365 (CAI is 42.4912 and MshEn is 58.6232), and reaction speed and tracking speed according to an anesthetic degree are also averagely 15 seconds faster than convention apparatuses. Furthermore, a correlation with a BIS apparatus in a stable state is high (0.9877), thereby showing an excellent result in terms of compatibility with an BIS analysis apparatus.

Although embodiments have been described with reference to a number of illustrative embodiments thereof, it should be understood that numerous other modifications and embodiments can be devised by those skilled in the art that will fall within the scope of the following claims.

**Claims**

1. A method for measuring an anesthetic depth, the method comprising:

    dividing, by an epoch dividing part, an EEG signal into multiple epoch signals on a time axis, extracting, by a counting part, a CAI calculation value (CAI) by counting the number of epoch signal points of a value higher than a determined critical value, extracting, by a Shannon entropy calculating part, a Shannon entropy calculation value (ShEn) by performing a Shannon entropy calculation from the EEG signal, and extracting, by a spectra entropy calculation part, a spectra entropy calculation value (SpEn) by performing a spectra entropy calculation;
    extracting, by a modified Shannon entropy calculating part, a modified Shannon entropy calculation value (MshEn) by multiplying the Shannon entropy calculation value (ShEn) and spectra entropy calculation value (SpEn); and
    extracting, by a CAI extracting part, an anesthetic depth index (MsCAI) by performing a logical operation on the modified Shannon entropy calculation value (MshEn) and CAI calculation value (CAI).

2. The method of claim 1, wherein the extracting of the anesthetic depth index (MsCAI) comprises extracting the anesthetic depth index (MsCAI) by multiplying the modified Shannon entropy calculation value (MshEn) by a first constant (L) and multiplying the CAI calculation value (CAI) by a second constant (U) to be summed up according to the equation MsCAI = L * MshEn + U * CAI (L = 0.243, $0.65 \leq U \leq 0.74$).

3. The method of claim 1, wherein the extracting of the spectra entropy calculation value (SpEn) comprises:

    performing low frequency band pass filtering on the EEG signal;
    performing high frequency band pass filtering on the signal obtained after the low frequency band pass filtering;
    generating a first epoch signal by dividing the signal obtained after the low frequency band pass filtering by predetermined time units;
    generating a second epoch signal by dividing the signal obtained after the high frequency band pass filtering by predetermined time units;
    removing noise from the first epoch signal; and
    performing normalization by dividing the second epoch signal by a root mean square value of the epoch signal having noise removed therefrom.

4. The method of claim 3, wherein the extracting of the spectra entropy calculation value (SpEn) further comprises:

    calculating a power spectrum density of a frequency component outputted by performing high speed Fourier transformation on the epoch signal with noise removed; and
    extracting the spectra entropy calculation value by performing a spectra entropy calculation from the power spectrum density.

5. The method of claim 4, further comprising extracting the Shannon entropy calculation value by performing a Shannon entropy calculation from the normalized signal.

6. The method of claim 5, further comprising performing high speed Fourier transformation on the normalized signal and extracting the spectra entropy calculation value the power spectrum density.

7. The method of claim 5, further comprising calculating the critical value by performing discrete Fourier transformation on the normalized signal to be multiplied at least twice, summed up to a predetermined frequency band, and multiplied by a constant.

8. The method of claim 7, further comprising extracting the CAI calculation value (CAI) by counting the number of points which are larger than the critical value, dividing the counted number by the total number of points of the epoch signal, and multiplying the divided value by a predetermined value.

9. An apparatus for measuring an anesthetic depth, the apparatus comprising:

a CAI extracting part configured to divide an EEG signal in a time section to generate an epoch signal, setting a predetermined critical value from the epoch signal, and extracting a cortical activity index (CAI) calculation value (CAI) by counting the number of points in an epoch signal exceeding the critical value;
a modified Shannon entropy extracting part configured to output a modified Shannon entropy calculation value (MshEn) by multiplying a Shannon entropy calculation value (ShEn) calculated from the EEG signal and a spectra entropy calculation value (SpEn) calculated from the EEG signal; and
an MsCAI extracting part configured to extract an anesthetic depth index (MsCAI) by combining the modified Shannon entropy calculation value (MshEn) and CAI calculation value (CAI).

10. The apparatus of claim 9, wherein the MsCAI extracting part extracts the anesthetic depth index (MsCAI) by multiplying the modified Shannon entropy calculation value (MshEn) by a first constant (L) and multiplying the CAI calculation value (CAI) by a second constant (U) to be summed up according to the equation MsCAI = L * MshEn + U * CAI (L = 0.243, $0.65 \leq U \leq 0.74$).

11. The apparatus of claim 10, wherein the CAI extracting part further comprises a critical value extracting part configured to fluidly change a constant multiplied by the critical value according to an anesthetic degree or a frequency band, so as to apply the anesthetic degree or frequency band to the critical value.

12. The apparatus of claim 11, wherein the critical value extracting part extracts the critical value by multiplying a value after discrete Fourier transformation performed on a normalized signal at least twice to be summed up to a predetermined frequency band and multiplied by a constant.

13. The apparatus of claim 9, wherein the CAI extracting part further comprises a counter configured to count the number of points which are larger than the critical value in the epoch signal and divide the counted number by the total number of points in the epoch signal.

14. The apparatus of claim 9, further comprising:

a first epoch dividing part configured to generate a first epoch signal by dividing a signal, which is obtained by performing low frequency band pass filtering on the EEG signal, by predetermined time units; and
a second epoch dividing part configured to generate a second epoch signal by performing high frequency band pass filtering on the signal obtained after the low frequency band pass filtering and by dividing the resultant signal by predetermined time units.

15. The apparatus of claim 14, further comprising:

a noise removing part configured to remove noise through a wavelet technique from an output of the first epoch dividing part;
a normalizing part configured to calculate a root mean square value of an output epoch signal of the noise removing part and divide an output epoch signal of the second epoch dividing part by the root mean square value; and
a Shannon entropy calculating part configured to calculate Shannon entropy from an output of the normalizing part so as to output the Shannon entropy calculation value.

16. The apparatus of claim 15, further comprising:

a power spectrum calculating part configured to perform high speed Fourier transformation on an output of the noise removing part and output a power spectrum density; and
a spectra entropy calculating part configured to output a spectra entropy calculation value by calculating spectra entropy from an output of the power spectrum calculating part.

17. A computer-readable recording medium, in which a computer program for performing the method for measuring an anesthetic depth of any one claim of claim 1 to claim 8 is stored.

**Patentansprüche**

1. Verfahren zum Messen einer Anästhesietiefe, wobei das Verfahren die Schritte aufweist:

Teilen eines EEG-Signals durch einen Zeitfensterteilungsabschnitt in mehrere Zeitfensersignale auf einer Zeitachse, Extrahieren eines CAI-Rechenwerts (CAI) durch Zählen der Anzahl von Zeitfenstersignalpunkten mit einem Wert, der höher ist als ein vorgegebener kritischer Wert, durch einen Zählabschnitt, Extrahieren eines Shannon-Entropie-Rechenwertes (ShEn) durch einen Shannon-Entropie-Berechnungsabschnitt durch Ausführen einer Shannon-Entropie-Berechnung bezüglich des EEG-Signals und Extrahieren eines Rechenwertes für eine spektrale Entropie (SpEn) durch Ausführen einer Berechnung für eine spektrale Entropie durch einen Abschnitt zum Berechnen einer spektralen Entropie;
Extrahieren eines modifizierten Shannon-Entropie-Rechenwertes (MshEn) durch Multiplizieren des Shannon-Entropie-Rechenwertes mit einem Rechenwert für eine spektrale Entropie (SpEn) durch einen Abschnitt zum Berechnen eines modifizierten Shannon-Entropie-Wertes; und
Extrahieren eines Anästhesietiefenindex (MsCAI) durch Ausführen einer logischen Operation bezüglich des modifizierten Shannon-Entropie-Rechenwertes (MshEn) und des CAI-Rechenwertes (CAI) durch einen CAI-Extraktionsabschnitt.

2. Verfahren nach Anspruch 1, wobei das Extrahieren des Anästhesietiefenindex (MsCAI) das Extrahieren des Anästhesietiefenindex (MsCAI) durch Multiplizieren des modifizierten Shannon-Entropie-Rechenwertes (MshEn) mit einer ersten Konstanten (L) und Multiplizieren des CAI-Rechenwertes (CAI) mit einer zweiten Konstante (U) und Aufsummieren gemäß der Gleichung MsCAI = L * MshEn + U * CAI (L = 0,243, $0,65 \leq U \leq 0,74$) aufweist.

3. Verfahren nach Anspruch 1, wobei das Extrahieren des Rechenwertes für die spektrale Entropie (SpEn) aufweist:

Ausführen einer Niederfrequenz-Bandpassfilterung bezüglich des EEG-Signals;
Ausführen einer Hochfrequenz-Bandpassfilterung bezüglich des nach der Niederfrequenz-Bandpassfilterung erhaltenen Signals;
Erzeugen eines ersten Zeitfenstersignals durch Teilen des nach der Niederfrequenz-Bandpassfilterung erhaltenen Signals durch vorgegebene Zeiteinheiten;
Erzeugen eines zweiten Zeitfenstersignals durch Teilen des nach der Hochfrequenz-Bandpassfilterung erhaltenen Signals durch vorgegebene Zeiteinheiten;
Entfernen von Rauschen vom ersten Zeitfenstersignal; und
Ausführen einer Normalisierung durch Teilen des zweiten Zeitfenstersignals durch einen quadratischen Mittelwert des Zeitfenstersignals, von dem das Rauschen entfernt wurde.

4. Verfahren nach Anspruch 3, wobei das Extrahieren des Rechenwertes für die spektrale Entropie (SpEn) ferner aufweist:

Berechnen einer spektralen Leistungsdichte einer Frequenzkomponente, die durch Ausführen einer Hochgeschwindigkeits-Fourier-Transformation bezüglich des Zeitfenstersignals ausgegeben wird, von dem das Rauschen entfernt ist; und
Extrahieren des Rechenwertes für die spektrale Entropie durch Berechnen der spektralen Entropie von der spektralen Leistungsdichte.

**5.** Verfahren nach Anspruch 4, ferner mit dem Extrahieren des Shannon-Entropie-Rechenwertes durch Ausführen einer Shannon-Entropie-Berechnung bezüglich des normalisierten Signals.

**6.** Verfahren nach Anspruch 5, ferner mit dem Ausführen einer Hochgeschwindigkeits-Fourier-Transformation bezüglich des normalisierten Signals und Extrahieren des Rechenwertes für die spektrale Entropie von der spektralen Leistungsdichte.

**7.** Verfahren nach Anspruch 5, ferner mit dem Berechnen des kritischen Wertes mindestens durch Quadrieren eines Wertes, der durch Ausführen einer diskreten Fourier-Transformation bezüglich des normalisierten Signals erhalten wird, Aufsummieren des Wertes zu einem vorgegebenen Frequenzband und Multiplizieren mit einer Konstante.

**8.** Verfahren nach Anspruch 7, ferner mit dem Extrahieren des CAI-Rechenwertes (CAI) durch Zählen der Anzahl von Punkten, die größer sind als der kritische Wert, Teilen der gezählten Anzahl durch die Gesamtanzahl von Punkten des Zeitfenstersignals und Multiplizieren des geteilten Wertes durch einen vorgegebenen Wert.

**9.** Vorrichtung zum Messen einer Anästhesietiefe, wobei die Vorrichtung aufweist:

einen CAI-Extraktionsabschnitt, der dafür konfiguriert ist, ein EEG-Signal in einen Zeitabschnitt zu teilen, um ein Zeitfenstersignal zu erzeugen, einen vorgegebenen kritischen Wert vom Zeitfenstersignal festzulegen und einen Rechenwert für einen kortikalen Aktivitätsindex (CAI) durch Zählen der Anzahl von Punkten in einem Zeitfenstersignal zu extrahieren, die den kritischen Wert überschreiten;
einen Abschnitt zum Extrahieren einer modifizierten Shannon-Entropie, der dafür konfiguriert ist, einen modifizierten Shannon-Entropie-Rechenwert (MshEn) durch Multiplizieren eines Shannon-Entropie-Rechenwertes (ShEn), der vom EEG-Signal berechnet wird, und eines Rechenwertes für die spektrale Entropie (SpEn) auszugeben; und
einen MsCAI-Extraktionsabschnitt, der dafür konfiguriert ist, einen Anästhesietiefenindex (MsCAI) durch Kombinieren des modifizierten Shannon-Entropie-Rechenwertes (MshEn) und des CAI-Rechenwerts (CAI) zu extrahieren.

**10.** Vorrichtung nach Anspruch 9, wobei der MsCAI-Extraktionsabschnitt den Anästhesietiefenindex (MsCAI) durch Multiplizieren des modifizierten Shannon-Entropie-Rechenwertes (MshEn) mit einer ersten Konstanten (L) und Multiplizieren des CAI-Rechenwerts (CAI) mit einer zweiten Konstanten (U) und Aufsummieren gemäß der Gleichung MsCAI = L * MshEn + U * CAI (L = 0,243, $0,65 \leq U \leq 0,74$) extrahiert.

**11.** Vorrichtung nach Anspruch 10, wobei der CAI-Extraktionsabschnitt ferner einen Abschnitt zum Extrahieren eines kritischen Wertes aufweist, der dafür konfiguriert ist, eine Konstante, die mit dem kritischen Wert multipliziert wird, gemäß einem Anästhesiegrad oder einem Frequenzband dynamisch zu ändern, um den Anästhesiegrad oder das Frequenzband auf den kritischen Wert anzuwenden.

**12.** Vorrichtung nach Anspruch 11, wobei der Abschnitt zum Extrahieren eines kritischen Wertes den kritischen Wert extrahiert durch mindestens Quadrieren eines Wertes, der durch eine diskrete Fourier-Transformation erhalten wird, die bezüglich eines normalisierten Signals ausgeführt wird, Aufsummieren des Wertes zu einem vorgegebenen Frequenzband und Multiplizieren mit einer Konstanten.

**13.** Vorrichtung nach Anspruch 9, wobei der CAI-Extraktionsabschnitt ferner einen Zähler aufweist, der dafür konfiguriert ist, die Anzahl von Punkten, die größer sind als der kritische Wert, im Zeitfenstersignal zu zählen, und die gezählte Anzahl durch die Gesamtzahl von Punkten im Zeitfenstersignal zu teilen.

**14.** Vorrichtung nach Anspruch 9, ferner mit:

einem ersten Zeitfensterteilungsabschnitt, der dafür konfiguriert ist, ein erstes Zeitfenstersignal durch Teilen eines Signals, das durch Ausführen einer Niederfrequenz-Bandpassfilterung bezüglich des EEG-Signals erhalten wird, durch vorgegebene Zeiteinheiten zu erzeugen; und
einen zweiten Zeitfensterteilungsabschnitt, der dafür konfiguriert ist, ein zweites Zeitfenstersignal durch Ausführen einer Hochfrequenz-Bandpassfilterung nach der Niederfrequenz-Bandpassfilterung und durch Teilen des erhaltenen Signals durch vorgegebene Zeiteinheiten zu erzeugen.

**15.** Vorrichtung nach Anspruch 14, ferner mit:

einem Rauschsignalentfernungsabschnitt, der dafür konfiguriert ist, Rauschen durch eine Wavelet-Technik von einem Ausgangssignal des ersten Zeitfensterteilungsabschnitts zu entfernen;

einem Normalisierungsabschnitt, der dafür konfiguriert ist, einen quadratischen Mittelwert eines Ausgangs-Zeitfenstersignals des Rauschentfernungsabschnitts zu berechnen und ein Ausgangs-Zeitfenstersignal des zweiten Zeitfensterteilungsabschnitts durch den quadratischen Mittelwert zu teilen; und

einem Shannon-Entropie-Berechnungsabschnitt, der dafür konfiguriert ist, eine Shannon-Entropie von einem Ausgangssignal des Normalisierungsabschnitts zu berechnen, um den Shannon-Entropie-Rechenwert auszugeben.

**16.** Vorrichtung nach Anspruch 15, ferner mit:

einem Abschnitt zum Berechnen einer spektralen Leistungsdichte, der dafür konfiguriert ist, eine Hochgeschwindigkeits-Fourier-Transformation bezüglich eines Ausgangssignals des Rauschsignalentfernungsabschnitts auszuführen und eine spektrale Leistungsdichte auszugeben; und

einem Abschnitt zum Berechnen einer spektralen Entropie, der dafür konfiguriert ist, einen Rechenwert für eine spektrale Entropie durch Berechnen einer spektralen Entropie von einem Ausgangssignal des Abschnitts zum Berechnen einer spektralen Leistungsdichte auszugeben.

**17.** Computerlesbares Speichermedium, auf dem ein Computerprogramm zum Ausführen des Verfahrens zum Messen einer Anästhesietiefe nach einem der Ansprüche 1 bis 8 gespeichert ist.

**Revendications**

**1.** Procédé pour la mesure d'une profondeur d'anesthésie, le procédé comprenant :

la division, par une partie de division de période, d'un signal EEG en une multitude de signaux de période sur un axe temporel, l'extraction, par une partie de comptage, d'une valeur de calcul CAI (CAI) en comptant le nombre de points de signal de période d'une valeur plus élevée qu'une valeur critique déterminée, l'extraction, par une partie de calcul d'entropie de Shannon, d'une valeur de calcul d'entropie de Shannon (ShEn) en effectuant un calcul d'entropie de Shannon à partir du signal EEG, et l'extraction, par une partie de calcul d'entropie spectrale, d'une valeur de calcul d'entropie spectrale (SpEn) en effectuant un calcul d'entropie spectrale ;

l'extraction, par une partie de calcul d'entropie de Shannon modifiée, d'une valeur de calcul d'entropie de Shannon modifiée (MshEn) en multipliant la valeur de calcul d'entropie de Shannon (ShEn) et la valeur de calcul d'entropie spectrale (SpEn) ; et

l'extraction, par une partie d'extraction CAI, d'un indice de profondeur d'anesthésie (MsCAI) en effectuant une opération logique sur la valeur de calcul d'entropie de Shannon modifiée (MshEn) et la valeur de calcul CAI (CAI).

**2.** Procédé selon la revendication 1, dans lequel l'extraction de l'indice de profondeur d'anesthésie (MsCAI) comprend l'extraction de l'indice de profondeur d'anesthésie (MsCAI) en multipliant la valeur de calcul d'entropie de Shannon modifiée (MshEn) par une première constante (L) et en multipliant la valeur de calcul CAI (CAI) par une deuxième constante (U) à additionner selon l'équation MsCAI = L * MshEn + U * CAI (L = 0,243, $0,65 \leq U \leq 0,74$).

**3.** Procédé selon la revendication 1, dans lequel l'extraction de la valeur de calcul d'entropie spectrale (SpEn) comprend :

l'exécution d'un filtrage passe-bande basse fréquence sur le signal EEG ;

l'exécution d'un filtrage passe-bande haute fréquence sur le signal obtenu après le filtrage passe-bande basse fréquence ;

la génération d'un premier signal de période en divisant le signal obtenu après le filtrage passe-bande basse fréquence par des unités de temps prédéterminées ;

la génération d'un deuxième signal de période en divisant le signal obtenu après le filtrage passe-bande haute fréquence par des unités de temps prédéterminées ;

l'élimination de bruit sur le premier signal de période ; et

l'exécution d'une normalisation en divisant le deuxième signal de période par une valeur quadratique moyenne du signal de période dont le bruit a été éliminé.

**4.** Procédé selon la revendication 3, dans lequel l'extraction de la valeur de calcul d'entropie spectrale (SpEn) comprend en outre :

le calcul d'une densité spectrale de puissance d'une composante de fréquence émise en exécutant une transformation de Fourier à grande vitesse sur le signal de période dont le bruit a été éliminé ; et
l'extraction de la valeur de calcul d'entropie spectrale en effectuant un calcul d'entropie spectrale à partir de la densité spectrale de puissance.

**5.** Procédé selon la revendication 4, comprenant en outre l'extraction d'une valeur de calcul d'entropie de Shannon en effectuant un calcul d'entropie de Shannon à partir du signal normalisé.

**6.** Procédé selon la revendication 5, comprenant en outre l'exécution d'une transformation de Fourier à grande vitesse sur le signal normalisé et l'extraction de la valeur de calcul d'entropie spectrale à partir de la densité spectrale de puissance.

**7.** Procédé selon la revendication 5, comprenant en outre le calcul d'une valeur critique en exécutant une transformation de Fourier discrète sur le signal normalisé à multiplier au moins deux fois, ajoutée à une bande de fréquence prédéterminée, et multipliée par une constante.

**8.** Procédé selon la revendication 7, comprenant en outre l'extraction de la valeur de calcul CAI (CAI) en comptant le nombre de points supérieurs à la valeur critique, en divisant le nombre compté par le nombre total de points du signal de période, et en multipliant la valeur divisée par une valeur prédéterminée.

**9.** Dispositif pour la mesure d'une profondeur d'anesthésie, le dispositif comprenant :

une partie d'extraction CAI configurée pour diviser un signal EEG en une section temporelle pour générer un signal de période, pour régler une valeur critique prédéterminée à partir du signal de période, et pour extraire une valeur de calcul (CAI) d'indice d'activité corticale (CAI) en comptant le nombre de points dans un signal de période dépassant la valeur critique ;
une partie d'extraction d'entropie de Shannon modifiée, configurée pour émettre une valeur de calcul d'entropie de Shannon modifiée (MshEn) en multipliant une valeur de calcul d'entropie de Shannon (ShEn) calculée à partir du signal EEG et une valeur de calcul d'entropie spectrale (SpEn) calculée à partir du signal EEG ; et
une partie d'extraction MsCAI configurée pour extraire un indice de profondeur d'anesthésie (MsCAI) en combinant la valeur de calcul d'entropie de Shannon modifiée (MshEn) et la valeur de calcul CAI (CAI).

**10.** Dispositif selon la revendication 9, dans lequel la partie d'extraction MsCAI extrait l'indice de profondeur d'anesthésie (MsCAI) en multipliant la valeur de calcul d'entropie de Shannon modifiée (MshEn) par une première constante (L) et en multipliant la valeur de calcul CAI (CAI) par une deuxième constante (U) à additionner selon l'équation MsCAI = L * MshEn + U * CAI (L = 0,243, $0{,}65 \leq U \leq 0{,}74$).

**11.** Dispositif selon la revendication 10, dans lequel la partie d'extraction CAI comprend en outre une partie d'extraction de valeur critique configurée pour modifier de façon fluide une constante multipliée par la valeur critique selon un degré d'anesthésie ou une bande de fréquence, de manière à appliquer le degré d'anesthésie ou la bande de fréquence à la valeur critique.

**12.** Dispositif selon la revendication 11, dans lequel la partie d'extraction de valeur critique extrait la valeur critique en multipliant une valeur après une transformation de Fourier discrète exécutée sur un signal normalisé au moins deux fois, à ajouter à une bande de fréquence prédéterminée et multipliée par une constante.

**13.** Dispositif selon la revendication 9, dans lequel la partie d'extraction CAI comprend en outre un compteur configuré pour compter le nombre de points supérieurs à la valeur critique dans le signal de période et pour diviser le nombre compté par le nombre total de points dans le signal de période.

**14.** Dispositif selon la revendication 9, comprenant en outre :

une première partie de division de période, configurée pour générer un premier signal de période en divisant un signal obtenu en exécutant un filtrage passe-bande basse fréquence sur le signal EEG, par des unités de temps prédéterminées ; et

une deuxième partie de division de période configurée pour générer un deuxième signal de période en exécutant un filtrage passe-bande haute fréquence sur le signal obtenu après le filtrage passe-bande basse fréquence et en divisant le signal résultant par des unités de temps prédéterminées.

**15.** Dispositif selon la revendication 14, comprenant en outre :

une partie d'élimination de bruit configurée pour éliminer du bruit par une technique d'ondelettes à partir d'une sortie de la première partie de division de période ;
une partie de normalisation configurée pour calculer une valeur quadratique moyenne d'un signal de période émis de la partie d'élimination de bruit et pour diviser un signal de période émis de la deuxième partie de division de période par la valeur quadratique moyenne ; et
une partie de calcul d'entropie de Shannon, configurée pour calculer une entropie de Shannon à partir d'une sortie de la partie de normalisation, de manière à émettre la valeur de calcul d'entropie de Shannon.

**16.** Dispositif selon la revendication 15, comprenant en outre :

une partie de calcul spectral de puissance, configurée pour exécuter une transformation de Fourier à grande vitesse sur une sortie de la partie d'élimination de bruit et pour émettre une densité spectrale de puissance ; et
une partie de calcul d'entropie spectrale, configurée pour émettre une valeur de calcul d'entropie spectrale en calculant une entropie spectrale à partir d'une sortie de la partie de calcul spectral de puissance.

**17.** Support d'enregistrement lisible par ordinateur, sur lequel est enregistré un programme informatique pour l'exécution du procédé pour la mesure d'une profondeur d'anesthésie selon l'une quelconque des revendications 1 à 8.

# FIG. 1

## FIG. 2

EEG SIGNAL

LOW FREQUENCY BAND PASS FILTER — 1

HIGH FREQUENCY BAND PASS FILTER — 7

FIRST EPOCH DIVIDING PART — 2

SECOND EPOCH DIVIDING PART — 8

NOISE REMOVING PART — 3

RMS

NORMALIZING PART — 9

POWER SPECTRUM CALCULATING PART — 4

SHANNON ENTROPY CALCULATING PART — 10

SPECTRA ENTROPY CALCULATING PART — 5

MshEn EXTRACTING PART — 11

MsCAI EXTRACTING PART — 14

ERROR REMOVING PART — 16

CRITICAL VALUE EXTRACTING PART — 6

CALCULATOR — 12

CAI EXTRACTING PART — 13

SCREEN DISPLAY PART — 17

DATA STORING PART — 19

## FIG. 3

## FIG. 4

**Inside Diameter Histogram**

## FIG. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- KR 20120131027

**Non-patent literature cited in the description**

- **ROGEAN RODRIGUES NUNES et al.** Spectral Entropy: A New Method for Anesthetic Adequacy. *Rev Bras Anestesiol,* vol. 54 (3), 402-422